# EUROPEAN PATENT APPLICATION

(11) **EP 4 144 410 A1**
(43) Date of publication of application: **08.03.2023**
(21) Application number: 22193177.7
(22) Date of filing: 31.08.2022
(51) Int. Cl.: A61N 1/378, A61N 1/36, A61N 1/05

(54) **ELECTRICAL STIMULATION DEVICE AND ELECTRICAL STIMULATION SYSTEM**

(30) Priority: 03.09.2021 CN 202111031703
(71) Applicant: Gimer Medical. Co., Ltd, New Taipei City 221 (TW)
(72) Inventor: PAN, Jian-Hao, New Taipei City (TW)
(74) Representative: Ter Meer Steinmeister & Partner

(57) **Abstract**

The present disclosure provides an electrical stimulation device. The electrical stimulation device includes a signal receiving circuit and a signal processing circuit. The signal receiving circuit receives and outputs a frequency signal. The signal processing circuit receives the frequency signal and provides an electrical stimulation signal according to the frequency signal.

## Description

This Application claims priority of China Patent Application No. 202111031703.7, filed on September 3, 2021.

### BACKGROUND

### Technology Field

The present disclosure relates to an electrical stimulation device, and, in particular, to an electrical stimulation device and an electrical stimulation system.

### Description of the Related Art

In recent years, dozens of therapeutic nerve electrical stimulation devices have been developed, and at least tens of thousands of people undergo electrical stimulation device implantation every year. Due to the development of precision manufacturing technology, the size of medical devices has been miniaturized and may be implanted inside the human body, for example, an implantable electrical stimulation device.

However, some of the current implantable electrical stimulation devices use an implantable electrical stimulator without batteries, instead using an external controller to provide electrical energy to the implantable electrical stimulator so that it can provide electrical stimulation in the manner of wireless power supply. The external controller and the implantable electrical stimulator without battery must be precisely aligned, otherwise the implantable electrical stimulator may not receive electrical energy. In addition, when the implantable electrical stimulator is implanted deep, the efficiency of the implantable electrical stimulator to receive electrical energy may be greatly decreased. Furthermore, the implantable electrical stimulator can easily be induced by mistake. As long as the implantable electrical stimulator is close to unspecified emission source with a similar electromagnetic wave, the implantable electrical stimulator may be immediately induced to perform electrical stimulation, thereby increasing the trouble of the user. Therefore, how to effectively improve the design of the implantable electrical stimulation device to decrease the complexity of circuit design, decrease the size of the electrical stimulation device and increase the convenience of use has become an important issue.

### SUMMARY

It is an object oof the present disclosure to provide an electrical stimulation device and an electrical stimulation system being decreased in the complexity of circuit design, decreased in size of the electrical stimulation device, and having an increased the convenience of use.

The object is solved by the features of the independent claims preferred embodiments are given in the dependent claims.

An embodiment of the present disclosure provides an electrical stimulation device, which includes a signal receiving circuit and a signal processing circuit. The signal receiving circuit is configured to receive and output a frequency signal. The signal processing circuit is configured to receive the frequency signal, and generate an electrical stimulation signal according to the frequency signal.

In one or more embodiments, the electrical stimulation device may further comprise at least one electrode, wherein the at least one electrode may be coupled to the signal processing circuit.

In one or more embodiments, the electrical stimulation device may not have a control unit.

In one or more embodiments, the electrical stimulation device may not have a battery.

In one or more embodiments, the signal processing circuit may be a passive filter.

In one or more embodiments, the signal processing circuit may comprise a first inductor, a first capacitor, a second inductor, a second capacitor, a third capacitor and a third inductor.

In one or more embodiments, the first inductor may comprise a first terminal and a second terminal, wherein the first terminal of the first inductor may be coupled to the signal receiving circuit and receives the frequency signal.

In one or more embodiments, the first capacitor may comprise a first terminal and a second terminal, wherein the first terminal of the first capacitor may be coupled to the second terminal of the first inductor.

In one or more embodiments, the second inductor may comprise a first terminal and a second terminal, wherein the first terminal of the second inductor may be coupled to the second terminal of the first capacitor, and the second terminal of the second inductor may be coupled to a ground terminal.

In one or more embodiments, the second capacitor may comprise a first terminal and a second terminal, wherein the first terminal of the second capacitor may be coupled to the first terminal of the second inductor, and the second terminal of the second capacitor may be coupled to the second terminal of the second inductor.

In one or more embodiments, the third capacitor may comprise a first terminal and a second terminal, wherein the first terminal of the third capacitor may be coupled to the second terminal of the first capacitor.

In one or more embodiments, the third inductor may comprise a first terminal and a second terminal, wherein the first terminal of the third inductor may be coupled to the second terminal of the third capacitor, and the second terminal of the third inductor may generate the electrical stimulation signal.

In one or more embodiments, the electrical stimulation device may further comprise a protection circuit, coupled to the signal processing circuit.

In one or more embodiments, the protection circuit may be configured to determine whether to output the electrical stimulation signal.

In one or more embodiments, the wherein the protection circuit may be a Hall switch or a magnetic reed switch.

In one or more embodiments, the electrical stimulation device may further comprise at least one electrode, wherein the at least one electrode is coupled to the protection circuit.

In one or more embodiments, the signal receiving circuit may comprise a coil, an antenna, an ultrasonic receiver, or a combination thereof.

In one or more embodiments, a frequency range of the frequency signal may comprise a range of 1MHz to 1.2MHz, a range of 9KHz to 200KHz, or a range of 6MHz to 45MHz.

In one or more embodiments, a frequency of the frequency signal may be 13.56MHz.

In one or more embodiments, a frequency range of the electrical simulation signal may be 100KHz to 2000KHz.

In one or more embodiments, a frequency range of the electrical simulation signal may be a range of 480KHz to 520KHz.

In one or more embodiments, a voltage range of the electrical stimulation signal may be between -25V and 25V.

In one or more embodiments, a current range of the electrical simulation signal may be between 0 and 60mA.

In one or more embodiments, a frequency of the frequency signal and a frequency of the electrical stimulation signal may be the same.

In addition, an embodiment of the present disclosure provides an electrical stimulation system, which includes an external controller and an electrical stimulation device. The external controller includes a signal generating circuit and a signal transmitting circuit. The signal generating circuit is configured to generate a frequency signal. The signal transmitting circuit is configured to receive and transmit the frequency signal. The electrical stimulation device includes a signal receiving circuit and a signal processing circuit. The signal receiving circuit is configured to receive and output the frequency signal. The signal processing circuit, configured to receive the frequency signal, and generate an electrical stimulation signal according to the frequency signal.

In one or more embodiments, the electrical stimulation device may further comprise a rectifying circuit, coupled between the signal receiving circuit and the signal processing circuit, and configured to receive the frequency signal and rectify the frequency signal to generate a rectified frequency signal.

In one or more embodiments, the electrical stimulation device may further comprise a protection circuit, coupled to the signal processing circuit, wherein the protection circuit is configured to determine whether to output the electrical stimulation signal.

In one or more embodiments, the protection circuit may be a Hall switch or a magnetic reed switch.

In one or more embodiments, each of the signal transmitting circuit and the signal receiving circuit may comprise a coil, an antenna, an ultrasonic receiver, or a combination thereof.

In one or more embodiments, a frequency range of the frequency signal may comprise a range of 1MHz to 1.2MHz, a range of 9KHz to 200KHz, or a range of 6MHz to 45MHz.

In one or more embodiments, a frequency range of the electrical simulation signal may be 100KHz to 2000KHz.

According to the electrical stimulation device and the electrical stimulation system disclosed by the present disclosure, the signal receiving circuit receives and outputs the frequency signal, and the signal processing circuit receives the frequency signal, and generates the electrical stimulation signal according to the frequency signal. Therefore, the complexity of circuit design may be effectively decreased, the size of the electrical stimulation device is decreased, and the convenience of use is increased.

### BRIEF DESCRIPTION OF THE DRAWINGS

The present disclosure can be more fully understood by reading the subsequent detailed description and examples with references made to the accompanying drawings, wherein:
FIG. 1 is a schematic view of an electrical stimulation system according to an embodiment of the present disclosure;
FIG. 2 is a schematic view of an electrical stimulation device according to an embodiment of the present disclosure;
FIG. 3 is a schematic view of an electrical stimulation device according to another embodiment of the present disclosure;
FIG. 4 is a waveform diagram of an electrical stimulation signal of an electrical stimulation device according to an embodiment of the present disclosure;
FIG. 5 is a schematic view of an electrical stimulation system according to another embodiment of the present disclosure; and
FIG. 6 is a schematic view of an electrical stimulation system according to another embodiment of the present disclosure.

### DETAILED DESCRIPTION OF THE DISCLOSURE

Technical terms of the present disclosure are based on general definition in the technical field of the present disclosure. If the present disclosure describes or explains one or some terms, definition of the terms is based on the description or explanation of the present disclosure. Each of the disclosed embodiments has one or more technical features. In possible implementation, a person skilled in the art would selectively implement all or some technical features of any embodiment of the present disclosure or selectively combine all or some technical features of the embodiments of the present disclosure.

In each of the following embodiments, the same reference number represents the same or a similar element or component.

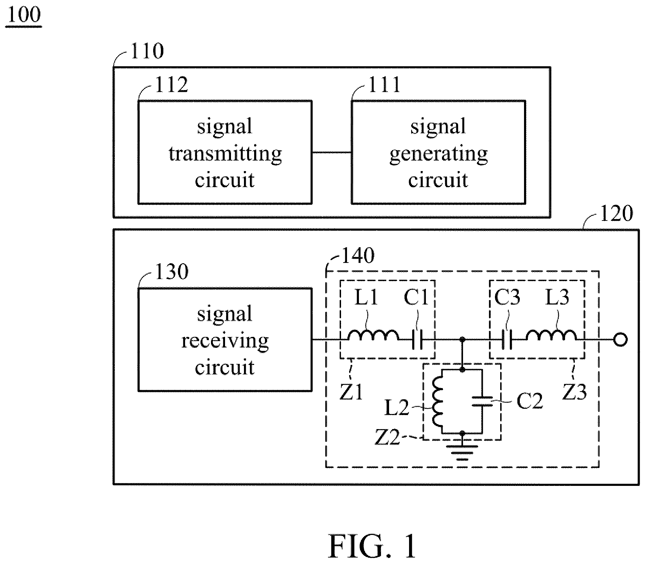

FIG. 1 is a schematic view of an electrical stimulation system according to an embodiment of the present disclosure. Please refer to FIG. 1. The electrical stimulation system 100 includes an external controller 110 and an electrical stimulation device 120. The external controller 110 at least includes a signal generating circuit 111 and a signal transmitting circuit 112. The signal generating circuit 111 is configured to generate a frequency signal. The signal transmitting circuit 112 is coupled to the signal generating circuit 111, and configured to transmit or transmit/receive the frequency signal. In some embodiments, the signal transmitting circuit 112 may include a coil, an antenna, an ultrasonic circuit, or a combination thereof, but the embodiment of the present disclosure is not limited thereto.

In the embodiment, the electrical stimulation device 120 is an implanted device and does not have a battery. That is, the electrical stimulation device 120 may be implanted inside the human body, and the external controller 110 may be configured outside the human body. Therefore, the electrical stimulation device 120 of the embodiment does not require any control unit, thereby decreasing the numbers of the circuit components in use and decreasing the complexity of circuit design. In an electrical stimulation device including a control unit, the control unit may be configured to perform frequency conversion of the received energy, control output time parameters (such as pulse width, pulse rate), etc. In addition, the external controller 110 may be configured at a position where the electrical stimulation device 120 may receive the frequency signal, and the external controller 110 does not need to be precisely aligned with the electrical stimulation device 120, thereby increasing the convenience of use.

The electrical stimulation device 120 includes a signal receiving circuit 130 and signal processing circuit 140. The signal receiving circuit 130 is configured to receive and output the frequency signal. In the embodiment, the signal receiving circuit 130 may include a coil, an antenna, an ultrasonic circuit, or a combination thereof, but the embodiment of the present disclosure is not limited thereto. For example, the signal receiving circuit 130 receives the frequency signal transmitted by the signal transmitting circuit 112 in a wireless manner, and output the received frequency signal. In some embodiments, when the signal transmitting circuit 112 and the signal receiving circuit 130 respectively include the coil, the energy transmission manner of the signal transmitting circuit 112 and the signal receiving circuit 130 may be an electromagnetic induction or an electromagnetic resonance. In some embodiments, when the signal transmitting circuit 112 and the signal receiving circuit 130 respectively include the antenna, the energy transmission manner of the signal transmitting circuit 112 and the signal receiving circuit 130 may be in the form of radio wave. In some embodiments, when the signal transmitting circuit 112 includes an ultrasonic generator and the signal receiving circuit 130 includes an ultrasonic transducer, the energy transmission manner of the signal transmitting circuit 112 and the signal receiving circuit 130 may be in the form of ultrasonic energy transmission.

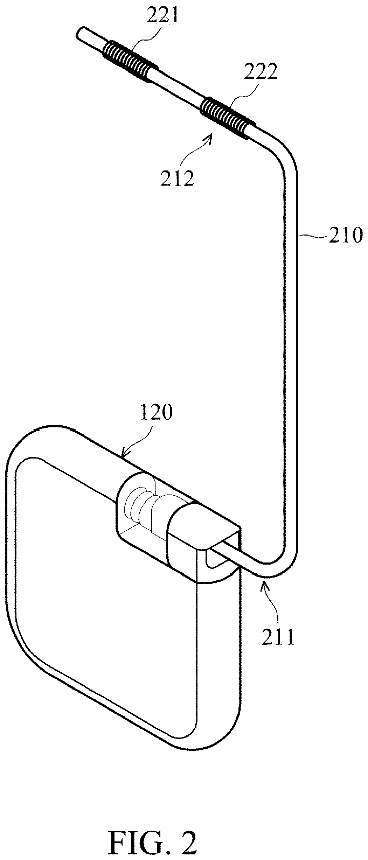

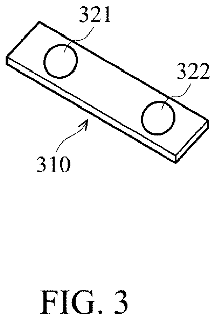

The signal processing circuit 140 is coupled to the signal receiving circuit 130. The signal processing circuit 140 is configured to receive the frequency signal, and generate an electrical stimulation signal according to the frequency signal. For example, in some embodiments, the signal processing circuit 140 may process (for example, filter and/or match) the frequency signal to generate the electrical stimulation signal. Then, the electrical stimulation signal may be output to the lead 210, such that the lead 210 may transmit the electrical stimulation signal a target area to be stimulated corresponding to an electrode 221 or an electrode 222 of the lead 210, so as to perform an electrical stimulation operation in the target area, as shown in FIG. 2. As shown in FIG. 3, the signal receiving circuit 130 and the signal processing circuit 140 in FIG. 1 may be configured in an electrical stimulation device 310 in a packaged manner, and an electrode 321 and an electrode 322 may be configured on one side of the electrical stimulation device 310. In some embodiment, in some embodiments, the signal processing circuit 140 may process (for example, filter and/or match) the frequency signal to generate the electrical stimulation signal. The electrical stimulation signal may be transmitted to a target area to be stimulated corresponding to the electrode 321 or the electrode 322, so as to perform an electrical stimulation operation in the target area. In some embodiments, the electrical stimulation device 120 is a flat rectangular parallelepiped with a length of 3 cm, a width of 1 cm, and a height of 0.5 cm. In addition, the electrical stimulation device 310 of FIG. 3 may be applied to nerves in the shallower layer of human tissue, such as the tibial nerve.

Furthermore, the signal processing circuit 140 is, for example, a passive filter. For example, the signal processing circuit 140 may include an impedance unit Z1, an impedance unit Z2 and an impedance unit Z3. The impedance unit Z1 includes a first terminal and a second terminal. The first terminal of the impedance unit Z1 is coupled to the signal receiving circuit 130 and receives the frequency signal. The impedance unit Z2 includes a first terminal and a second terminal. The first terminal of the impedance unit Z2 is coupled to the second terminal of the impedance unit Z1. The second terminal of the impedance unit Z2 is coupled to a ground terminal. The impedance unit Z3 includes a first terminal and a second terminal. The first terminal of the impedance unit Z3 is coupled to the second terminal of the impedance unit Z1. The second terminal of the impedance unit Z3 generates the electrical stimulation signal.

Furthermore, the impedance unit Z1 may include an inductor L1 and a capacitor C1. The inductor L1 includes a first terminal and a second terminal. The first terminal of the inductor L1 serves as the first terminal of the impedance unit Z1, is coupled to the signal receiving circuit 130 and receives the frequency signal. The capacitor C1 includes a first terminal and a second terminal. The first terminal of the capacitor C1 is coupled to the second terminal of the inductor L1. The second terminal of the capacitor C1 serves as the second terminal of the impedance unit Z1.

The impedance unit Z2 may include an inductor L2 and a capacitor C2. The inductor L2 includes a first terminal and a second terminal. The first terminal of the inductor L2 serves as the first terminal of the impedance unit Z2, and is coupled to the second terminal of the capacitor C1. The second terminal of the inductor L2 serves as the second terminal of the impedance unit Z2, and is coupled to the ground. The capacitor C2 includes a first terminal and a second terminal. The first terminal of the capacitor C2 is coupled to the first terminal of the inductor L2. The second terminal of the capacitor C2 is coupled to the second terminal of the inductor L2. The impedance unit Z3 may include a capacitor C3 and an inductor L3. The capacitor C3 includes a first terminal and a second terminal. The first terminal of the capacitor C3 serves as the first terminal of the impedance unit Z3, and is coupled to the second terminal of the capacitor C1. The inductor L3 includes a first terminal and a second terminal. The first terminal of the inductor L3 is coupled to the second terminal of the capacitor C3. The second terminal of the inductor L3 serves as the second terminal of the impedance unit Z3, and generates the electrical stimulation signal.

In some embodiments, the frequency range of the above electrical stimulation signal is, for example, 100KHz to 2000KHz. In some embodiments, the frequency range of the electrical stimulation signal is, for example, 200KHz to 800KHz. In some embodiments, the frequency range of the electrical stimulation signal is, for example, 480KHz to 520KHz. In some embodiments, the frequency of the electrical stimulation signal is, for example, 500KHz. In addition, the above electrical stimulation signal is, for example, a sine wave signal. Furthermore, in the embodiment, the frequency of the electrical stimulation signal and the frequency of the frequency signal may be the same. Moreover, in addition to frequencies and waveforms of the frequency signal (i.e., the energy transmission signal) and the electrical stimulation signal being the same, the amplitudes, pulse bandwidths and pulse repetition frequencies of the frequency signal and the electrical stimulation signal may also be the same, but the embodiment of the present disclosure is not limited thereto.

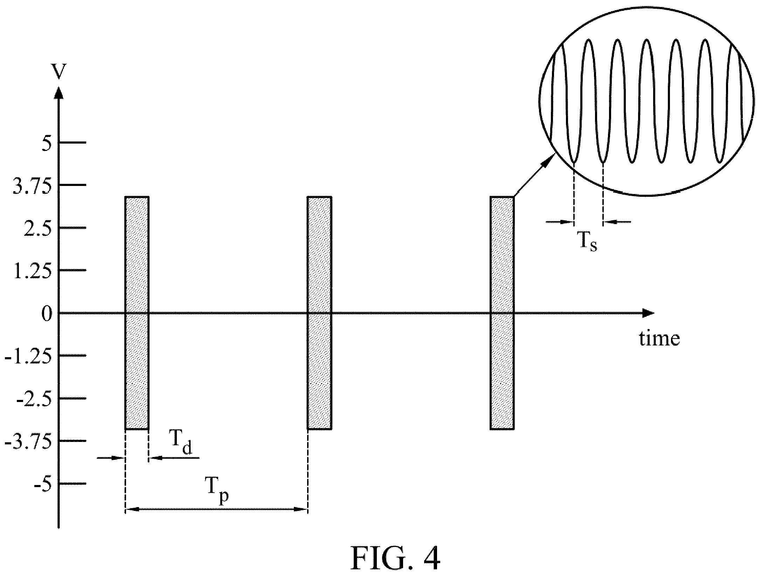

In some embodiments, as shown in FIG. 4, the above electrical stimulation signal may be a pulsed radio-frequency (PRF) signal (or referred to as a pulse signal), a continuous sine wave, a continuous triangular wave, etc., but the embodiment of the present disclosure is not limited thereto. In addition, when the electrical stimulation signal is a pulse alternating signal, one pulse cycle time Tₚ includes a plurality of pulse signals and at least one rest period of time, and the pulse cycle time Tₚ is the reciprocal of the pulse repetition frequency. The pulse repetition frequency range (also referred to as the pulse frequency range) is, for example, between 0 and 1KHz, preferably between 1 and 100Hz. In the embodiment, the pulse repetition frequency of the electrical stimulation signal is, for example, 2Hz. In addition, the duration time T_{d} of the plurality of pulses in one pulse cycle time is, for example, between 1 and 250 milliseconds (ms), preferably between 10 and 100ms. In the embodiment, the duration time T_{d} is, for example, 25ms. In the embodiment, the frequency of the electrical stimulation signal is 500KHz, in other words, the cycle time Tₛ of the electrical stimulation signal is about 2 microseconds (µs). Furthermore, the frequency of the above electrical stimulation signal is the intra-pulse frequency in each pulse alternating signal of FIG. 4. Moreover, the voltage range of the above electrical stimulation signal may be between -25V and 25V. Furthermore, the voltage range of the above electrical stimulation signal may further be between -20V and 20V. The current range of the above electrical stimulation signal may be between 0 and 60mA. Furthermore, the current range of the above electrical stimulation signal may further be between 0 and 50mA.

Furthermore, in the embodiment, the external controller 110 may be configured outside the human body, and the electrical stimulation device 120 may be implanted inside the human body. When the electrical stimulation device 120 is implanted inside the human body, the electrical stimulation device 120 may be placed under the skin of the human body, and one terminal 211 of the lead 210 is connected to the electrical stimulation device 120, and the other terminal 212 of the lead 210 is placed close to a target area to be stimulated. The electrical stimulation system 100 as a spinal cord electrical stimulation system is taken as an example, the other terminal 212 of at least part of the lead 210 is disposed in the epidural space to electrically stimulate the spinal cord, the spinal nerve or the dorsal root ganglia (DRG). The signal processing circuit 140 of the electrical stimulation device 120 transmits the electrical stimulation signal to an output electrode. That is, the signal processing circuit 140 may transmit the electrical stimulation signal to the output electrode (such as the electrode 221 or the electrode 222) of the other terminal 212 of the lead 210 through the terminal 211 of the lead 210, so as to electrically stimulate the target area. The current transmitted by the electrical stimulation device 120 may flow out from one output electrode (such as the electrode 221 or the electrode 222) of the lead 210, and then conducts through the human tissue, and then flow back to the lead 210 from the other electrode (such as the electrode 222 or the electrode 221). In the embodiment, the electrode 221 and the electrode 222 may be a pair of electrodes. In some embodiments, the electrode 221 may be a positive electrode, and the electrode 222 may be a negative electrode. In some embodiments, the electrode 221 may be the negative electrode, and the electrode 222 may be the positive electrode.

In addition, the target nerve area of electrical stimulation may also be in the brain for electrical stimulation of brain cortex or deep brain stimulation (DBS) or abdominal and peripheral nerves. In some embodiments, the electrical stimulation system 100 may also be used to relieve or treat diseases, such as pain, overactive bladder (OAB) , renal hypertension, spasm, premature ejaculation or carpal tunnel syndrome (CTS), etc. In some embodiments, the target nerve area of electrical stimulation may also be a lateral recess or a peripheral nervous system (PNS). Furthermore, the target area of the electrical stimulation may also receive less energy per unit time to ensure the subthreshold stimulation, so that the patient implanted with the electrical-stimulation system may reduce the chance of feeling paresthesia to perform the paresthesia-free treatment.

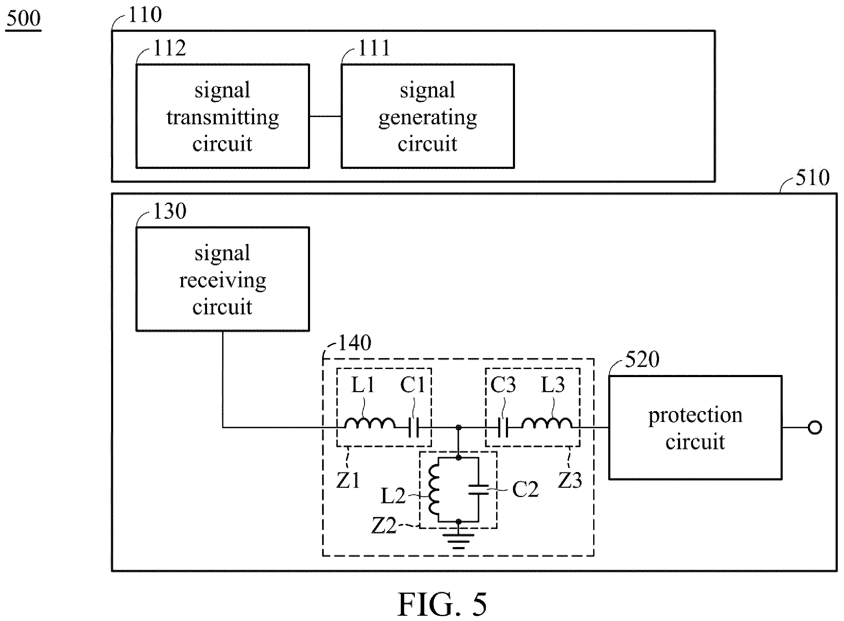

FIG. 5 is a schematic view of an electrical stimulation system according to another embodiment of the present disclosure. Please refer to FIG. 5. The electrical stimulation system 500 includes an external controller 110 and an electrical stimulation device 510. In the embodiment, the external controller 110 in FIG. 5 is the same as or similar to external controller 110 in FIG. 1. Accordingly, the external controller 110 in FIG. 5 may refer to the description of the embodiment of FIG. 1, and the description thereof is not repeated herein.

The electrical stimulation device 510 includes a signal receiving circuit 130, a signal processing circuit and a protection circuit 520. In the embodiment, the signal receiving circuit 130 and the signal processing circuit 140 in FIG. 5 are the same as or similar to the signal receiving circuit 130 and the signal processing circuit 140 in FIG. 1. Accordingly, the signal receiving circuit 130 and the signal processing circuit 140 in FIG. 5 may refer to the embodiment of FIG. 1, and the description thereof is not repeated herein.

The protection circuit 520 is coupled to the signal processing circuit 140 (such as the second terminal of the impedance unit Z3), and the protection circuit 520 is configured to determine whether to output the electrical stimulation signal generated by the signal processing circuit 140 (such as the second terminal of the impedance unit Z3). In some embodiments, the protection circuit 520 is, for example, a Hall switch or a magnetic reed switch, but the embodiment of the present disclosure is not limited thereto. For example, when the external controller 110 is configured with a triggering component (such as a magnet), the protection circuit 520 (such as the Hall switch or the magnetic reed switch) may sense the magnetic field generated by the triggering component (such as the magnet) and be turned on, so as to output the electrical stimulation signal. In addition, when the external controller 110 is not configured with a triggering component (such as a magnet), the protection circuit 520 (such as the Hall switch or the magnetic reed switch) may not sense the triggering component and not be turned on, and the protection circuit 520 does not output the electrical stimulation signal. Therefore, the situation that the electrical stimulation device 510 senses or receives the frequency signal generated by the other devices or emission sources to generate the error operation (for example, directly perform the electrical stimulation operation) may be effectively avoided, so as to increase the safety of the electrical stimulation device 510 and decrease the trouble of the user.

In addition, the signal receiving circuit 130, the signal processing circuit 140 and the protection circuit 520 may also be configured in the electrical stimulation device 310 of FIG. 3. The electrical stimulation signal generated by the signal processing circuit 140 may be transmitted to a target area to be stimulated corresponding to the electrode 321 or the electrode 322 through the protection circuit 520, so as to perform an electrical stimulation operation in the target area.

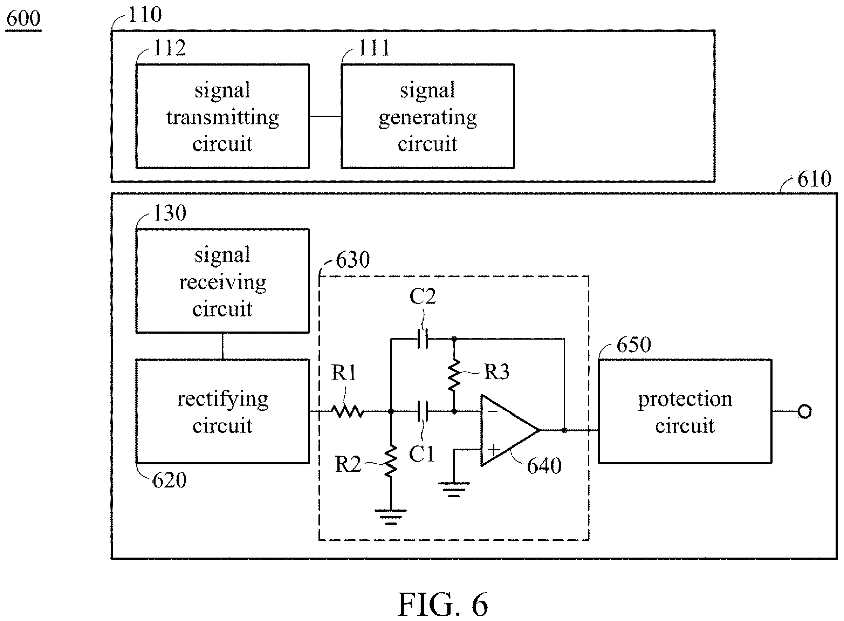

FIG. 6 is a schematic view of an electrical stimulation system according to another embodiment of the present disclosure. Please refer to FIG. 6. The electrical stimulation system 600 includes an external controller 110 and an electrical stimulation device 610. In the embodiment, the external controller 110 in FIG. 6 is the same as or similar to external controller 110 in FIG. 1. Accordingly, the external controller 110 in FIG. 6 may refer to the description of the embodiment of FIG. 1, and the description thereof is not repeated herein.

The electrical stimulation device 610 includes a signal receiving circuit 130, a rectifying circuit 620 and a signal processing circuit 630. In the embodiment, the signal receiving circuit 130 in FIG. 6 is the same as or similar to the signal receiving circuit 130 in FIG. 1. Accordingly, the signal receiving circuit 130 in FIG. 6 may refer to the embodiment of FIG. 1, and the description thereof is not repeated herein.

The rectifying circuit 620 is coupled to the signal receiving circuit 130, and configured to receive the frequency signal and rectify the frequency signal to generate a rectified frequency signal. In the embodiment, the rectifying circuit 620 may be a bridge rectifier, such as a half-bridge rectifier or a full-bridge rectifier, but the embodiment of the present disclosure is not limited thereto. In addition, the above rectified frequency signal is, for example, a square wave signal. The rectified frequency signal with the square wave signal may be processed through the signal processing circuit 630 to generate an electrical stimulation signal with a sine wave signal. In the above embodiment, the electrical stimulation signal is the square wave signal as an example, but the embodiment of the present disclosure is not limited thereto. In some embodiments, the electrical stimulation signal may also be a triangular wave, a pulse wave, etc.

The signal processing circuit 630 is coupled to the rectifying circuit 620. That is, the rectifying circuit 620 is coupled between the signal receiving circuit 130 and the signal processing circuit 630. The signal processing circuit 630 receives the frequency signal (such as the rectified frequency signal) and generates the electrical stimulation signal according to the frequency signal. For example, in some embodiments, the signal processing circuit 630 is similar to the signal processing circuit 140. The signal processing circuit 630 may process (for example, filter and/or match) the frequency signal to generate the electrical stimulation signal. Then, the electrical stimulation signal may be output to the lead 210, such that the lead 210 may transmit the electrical stimulation signal a target area to be stimulated corresponding to the electrode 221 or the electrode 222 of the lead 210, so as to perform an electrical stimulation operation in the target area, as shown in FIG. 2. In some embodiments, as shown in FIG. 3, the signal receiving circuit 130, the rectifying circuit 620, the signal processing circuit 630 and the protection circuit 650 in FIG. 6 may be configured in the electrical stimulation device 310 in a packaged manner. The signal processing circuit 630 may process (for example, filter and/or match) the frequency signal to generate the electrical stimulation signal. The electrical stimulation signal may be transmitted to a target area to be stimulated corresponding to the electrode 321 or the electrode 322 through the protection circuit 650, so as to perform an electrical stimulation operation in the target area.

Furthermore, the signal processing circuit 630 may be an active filter. For example, the signal processing circuit 630 may include a resistor R1, a resistor R2, a capacitor C4, a capacitor C5, a resistor R3 and an amplifier 640.

The resistor R1 includes a first terminal and a second terminal. The first terminal of the resistor R1 is coupled to the rectifying circuit 620, and receives the rectified frequency signal. The resistorR2 includes a first terminal and a second terminal. The first terminal of the resistor R2 is coupled to the second terminal of the resistor R1. The second terminal of the resistor R2 is coupled to the ground terminal.

The capacitor C4 includes a first terminal and a second terminal. The first terminal of the capacitor C4 is coupled to the second terminal of the resistor R1. The capacitor C5 includes a first terminal and a second terminal. The first terminal of the capacitor C5 is coupled to the first terminal of the capacitor C4. The resistor R3 includes a first terminal and a second terminal. The first terminal of the resistor R3 is coupled to the second terminal of the capacitor C4. The second terminal of the resistor R3 is coupled to the second terminal of the capacitor C5.

The amplifier 640 includes a first input terminal, a second input terminal and an output terminal. The first input terminal (such as a positive input terminal) of the amplifier 640 is coupled to the ground terminal. The second input terminal (such as a negative input terminal of the amplifier 640 is coupled to the second terminal of the resistor R3. The output terminal of the amplifier 640 is coupled to the first terminal of the resistor R3, and outputs the electrical stimulation signal.

In some embodiment, the frequency range of the above electrical stimulation signal is, for example, 100KHz to 2000KHz. In some embodiments, the frequency range of the electrical stimulation signal is, for example, 200KHz to 800KHz. In some embodiments, the frequency range of the electrical stimulation signal is, for example, 480KHz to 520KHz. In addition, the above electrical stimulation signal is, for example, a sine wave signal. Furthermore, in the embodiment, the frequency of the electrical stimulation signal and the frequency of the frequency signal may be the same. In some embodiments, the frequency of the electrical stimulation signal and the frequency of the frequency signal must be the same. Moreover, in addition to the frequencies and waveforms of the frequency signal (i.e., the energy transmission signal) and the electrical stimulation signal being the same, the amplitudes, pulse bandwidths and pulse repetition frequencies of the frequency signal and the electrical stimulation signal may also be the same, but the embodiment of the present disclosure is not limited thereto.

In the embodiment, the electrical stimulation device 610 further includes a protection circuit 650. The protection circuit 650 is coupled to the signal processing circuit 630 (such as the output terminal of the amplifier 640), and the protection circuit 650 is configured to determine whether to output the electrical stimulation signal generated by the signal processing circuit 630 (such as the output terminal of the amplifier 640). In some embodiments, the protection circuit 650 is, for example, the Hall switch or the magnetic reed switch, but the embodiment of the present disclosure is not limited thereto. For example, when the external controller 110 is configured with a triggering component (such as a magnet), the protection circuit 650 (such as the Hall switch or the magnetic reed switch) may sense the magnetic field generated by the triggering component (such as the magnet) and be turned on, so as to output the electrical stimulation signal. In addition, when the external controller 110 is not configured with a triggering component (such as a magnet), the protection circuit 650 (such as the Hall switch or the magnetic reed switch) may not sense the triggering component and not be turned on, and the protection circuit 650 does not output the electrical stimulation signal. Therefore, the situation that the electrical stimulation device 610 senses or receives the frequency signal generated by the other devices or emission sources to generate the error operation (for example, directly perform the electrical stimulation operation) may be effectively avoided, so as to increase the safety of the electrical stimulation device 610 and decrease the trouble of the user.

In summary, according to the electrical stimulation device and the electrical stimulation system disclosed by the present disclosure, the signal receiving circuit receives and outputs the frequency signal, and the signal processing circuit receives the frequency signal, and generates the electrical stimulation signal according to the frequency signal. Therefore, the complexity of circuit design may be effectively decreased, the size of the electrical stimulation device is decreased, and the convenience of use is increased.

In addition, the electrical stimulation device of the embodiment of the present disclosure further includes the protection circuit. The protection circuit may be coupled to the signal processing circuit, and is configured to determine whether to output the electrical stimulation signal generated by the signal processing circuit, wherein the electrical stimulation signal is a sine wave signal. Therefore, the situation that the electrical stimulation device senses or receives the frequency signal generated by the other devices or emission sources to generate the error operation (for example, directly perform the electrical stimulation operation) may be effectively avoided, so as to increase the safety of the electrical stimulation device and decrease the trouble of the user.

While the disclosure has been described by way of example and in terms of the preferred embodiments, it should be understood that the disclosure is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. An electrical stimulation device (120), comprising:
a signal receiving circuit (130), configured to receive and output a frequency signal; and
a signal processing circuit (140), configured to receive the frequency signal, and generate an electrical stimulation signal according to the frequency signal.

2. The electrical stimulation device as claimed in claim 1, further comprising at least one electrode (221, 222), wherein the at least one electrode (221, 222) is coupled to the signal processing circuit (140).

3. The electrical stimulation device as claimed in claim 1 or 2 , wherein the electrical stimulation device (120) does not have a control unit and/or the electrical stimulation device (120) does not have a battery.

4. The electrical stimulation device as claimed in any one of the preceding claims, wherein the signal processing circuit (140) is a passive filter.

5. The electrical stimulation device as claimed in any one of the preceding claims, wherein the signal processing circuit (140) comprises:
a first inductor (L1), comprising a first terminal and a second terminal, wherein the first terminal of the first inductor is coupled to the signal receiving circuit and receives the frequency signal;
a first capacitor (C1), comprising a first terminal and a second terminal, wherein the first terminal of the first capacitor is coupled to the second terminal of the first inductor;
a second inductor (L2), comprising a first terminal and a second terminal, wherein the first terminal of the second inductor is coupled to the second terminal of the first capacitor, and the second terminal of the second inductor is coupled to a ground terminal;
a second capacitor (C2), comprising a first terminal and a second terminal, wherein the first terminal of the second capacitor is coupled to the first terminal of the second inductor, and the second terminal of the second capacitor is coupled to the second terminal of the second inductor;
a third capacitor (C3), comprising a first terminal and a second terminal, wherein the first terminal of the third capacitor is coupled to the second terminal of the first capacitor; and
a third inductor (L3), comprising a first terminal and a second terminal, wherein the first terminal of the third inductor is coupled to the second terminal of the third capacitor, and the second terminal of the third inductor generates the electrical stimulation signal.

6. The electrical stimulation device as claimed in any one of the preceding claims, further comprising a protection circuit (520), coupled to the signal processing circuit (140, 630), wherein the protection circuit (520) is configured to determine whether to output the electrical stimulation signal.

7. The electrical stimulation device as claimed in claim 6, wherein the protection circuit (520) is a Hall switch or a magnetic reed switch.

8. The electrical stimulation device as claimed in claim 6 or 7, further comprising at least one electrode (221, 222), wherein the at least one electrode (221, 222) is coupled to the protection circuit (520).

9. The electrical stimulation device as claimed in any one of the preceding claims, wherein the signal receiving circuit (120) comprises a coil, an antenna, an ultrasonic receiver, or a combination thereof.

10. The electrical stimulation device as claimed in any one of the preceding claims, wherein a frequency range of the frequency signal comprises a range of 1MHz to 1.2MHz, a range of 9KHz to 200KHz, or a range of 6MHz to 45MHz; and/or a frequency of the frequency signal is 13.56MHz.

11. The electrical stimulation device as claimed in any one of the preceding claims, wherein a frequency range of the electrical simulation signal is 100KHz to 2000KHz and/or a frequency range of the electrical simulation signal is a range of 480KHz to 520KHz and/or a voltage range of the electrical stimulation signal is between -25V and 25V and/or a current range of the electrical simulation signal is between 0 and 60mA.

12. The electrical stimulation device as claimed in any one of the preceding claims, wherein a frequency of the frequency signal and a frequency of the electrical stimulation signal are the same.

13. An electrical stimulation system, comprising an external controller (110), comprising:
a signal generating circuit (111), configured to generate a frequency signal; and
a signal transmitting circuit (112), configured to receive and transmit the frequency signal; and
an electrical stimulation device (510) as claimed in any one of the preceding claims.

14. The electrical stimulation system as claimed in claim 13, wherein the electrical stimulation device (510) further comprises a rectifying circuit (620), coupled between the signal receiving circuit (130) and the signal processing circuit (630), and configured to receive the frequency signal and rectify the frequency signal to generate a rectified frequency signal.

15. The electrical stimulation system as claimed in claim 13, wherein each of the signal transmitting circuit (112) and the signal receiving circuit (130) comprises a coil, an antenna, an ultrasonic receiver, or a combination thereof.
